# EUROPÄISCHE PATENTANMELDUNG

(11) **EP 2 952 224 A1**
(43) Veröffentlichungstag der Anmeldung: **09.12.2015**
(21) Anmeldenummer: 15164573.6
(22) Anmeldetag: 22.04.2015
(51) Int. Cl.: A61N 1/08, A61N 1/37, G01R 33/022, G01R 33/24, G01R 33/28

(54) **DETEKTOR FÜR ELEKTROMAGNETISCHE FELDER**

(30) Priorität: 05.06.2014 US 201462007953 P
(71) Anmelder: BIOTRONIK SE & Co. KG, 12359 Berlin (DE)
(72) Erfinder: Dörr, Thomas, 12437 Berlin (DE); Kibbel, Steffen, 10559 Berlin (DE); Weiss, Ingo, 12435 Berlin (DE)
(74) Vertreter: Galander, Marcus

(57) **Zusammenfassung**

IMD umfassend mindestens eine Energieversorgung, mindestens eine Abfühlvorrichtung und/oder mindestens einer Stimulationsvorrichtung, mindestens eine Kontrolleinheit; mindestens eine MR-Detektionseinheit, und mindestens zwei Magnetfeldsensoren, wobei die mindestens eine Energieversorgung mit einem oder mehreren der anderen Komponenten direkt oder indirekt verbunden ist, und wobei die mindestens eine Kontrolleinheit mit der mindestens einen Abfühlvorrichtung und/oder mindestens einen Stimulationsvorrichtung direkt oder indirekt verbunden ist, und die mindestens eine Kontrolleinheit mit der mindestens einen MR-Detektionseinheit und dem mindestens einen Magnetfeldsensor direkt oder indirekt verbunden ist, dadurch gekennzeichnet, dass die zwei Magnetfeldsensoren räumlich von einander getrennt platziert sind, und die MR-Detektionseinheit ausgebildet ist aus den von den Magnetfeldsensoren detektierten und an die MR-Detektionseinheit übermittelten Magnetfeldstärken einen räumlichen und/oder zeitlichen Gradienten der Magnetfeldstärken zu bestimmen, und ein MR-Feld zu detektieren und ein MR-Signal an die Kontrolleinheit zu übermitteln.

## Beschreibung

Die Erfindung bezieht sich auf ein Verfahren zur Erkennung von elektromagnetischen Feldern, speziell auf solche, wie sie bei bildgebenden Kernspintomographie- (MRI-, MRT-) Geräten auftreten, für implantierbare medizinische Geräte.

Obwohl MR-/MRI-Untersuchungen (MR-, MRT und MRI- werden im Folgenden unter MR- subsumiert) einen immer höheren Stellenwert in der diagnostischen Medizin erhalten, ist ein Teil der Patienten für MR-Untersuchungen kontraindiziert. Eine solche Kontraindizierung kann durch ein aktives implantiertes medizinisches Gerät gegeben sein.

Problematisch sind neben möglichen Erwärmungseffekten von Implantaten, besonders bei kleinen leitenden Strukturen, fehlerhafte Erkennungen von Ereignissen im Herzen, wie aber nicht ausschließlich Kammerflimmern, bzw. schnelle Herzrhythmusstörungen, (VF) und die hohen statischen Magnetfelder und daraus resultierende Magnetisierungen von elektrischen Komponenten. Besonders bei aktiven Implantaten wie aber nicht ausschließlich Defibrillatoren/Kardioverter (ICD), Schrittmachern, kardialen Resynchronisationsgeräten, Neurostimulatoren oder Medikamentenpumpen, aber auch bei passiven Implantaten wie Monitoringgeräten treten oben genannte Effekte auf. Aber auch andere implantierten medizinischen Geräten können durch elektromagnetische Felder in Ihrer Funktion gestört werden und dürfen nicht in Umgebungen mit erhöhten elektromagnetischen Belastungen betrieben werden. Der folgende Stand der Technik geht vor allem auf die Problematik der Detektion von elektromagnetischen Störfeldern bei implantierten medizinischen Geräten (IMD) ein.
Die US 6,522,920 beschreibt ein System zum Schutz der induktiv angesteuerten Hochspannungsschalter eines ICD bei der Schockabgabe in einem Magnetfeld. Dazu wird überwacht, ob eine ausreichende Gatespannung zum Zeitpunkt der Schockabgabe bereit steht. Die Überwachung wird durch eine Beurteilung der Sekundärspannung der induktiven Treiber beurteilt.

Aus der US 2008/0071168 ist bekannt, zur Detektion eines Magnetfeldes eine Impedanzmesseinheit und ein RLC-Glied vorzusehen und die Detektion durch Bestimmung der Induktivität in der RLC-Komponente vorzunehmen. Dafür wird eine zusätzliche oder angepasste Impedanzmesseinheit benötigt.

Des Weiteren beschreibt die US 7,509,167 die Erkennung eines Magnetfeldes durch die Messung der Steuerzeiten für die Ansteuerung des Hochspannungstransformators der Primärseite oder alternativ die Messung von Spitzenströmen während eines Ladezyklus. Nachteil des beschriebenen Systems ist, dass die Messung der Steuerzeiten sehr schnell sein muss (µs bis ns), bzw. dass eine direkte Strommessung aufgrund des notwendigen Messwiderstandes, der die Ladezeiten für den Hochspannungskondensator verlängern würde, nur schwer implementierbar ist.

Des Weiteren beschreibt die US 6,101,417 die Verwendung eines GMR-Sensors anstelle eines Read-Switches, allerdings nicht zur Detektion von MR-Feldern, sonder zur Aktivierung eines Magnetmodus im Implantat.

Die aus dem Stand der Technik bekannten technischen Lösungen zur Detektion von elektromagnetischen Störfeldern bei implantierten medizinischen Geräten weisen aber Unsicherheiten hinsichtlich der Sensitivität oder Spezifität auf oder sind nicht ausreichend effizient.

Daher ist es Aufgabe der Erfindung, eine effiziente und zuverlässige Vorrichtung zur MR-Detektion bereitzustellen.

Die Aufgabe wird durch ein implantierbares medizinisches Gerät (IMD) mit den Merkmalen des Anspruchs 1 gelöst.
Ein solches IMD umfasst mindestens eine Energieversorgung,
mindestens eine Abfühlvorrichtung und/oder mindestens eine Stimulationsvorrichtung,
mindestens eine Kontrolleinheit;
mindestens eine MR-Detektionseinheit, und
mindestens zwei Magnetfeldsensoren, wobei
die mindestens eine Energieversorgung mit einem oder mehreren der anderen Komponenten direkt oder indirekt verbunden ist, und wobei
die mindestens eine Kontrolleinheit mit der mindestens einen Abfühlvorrichtung und/oder mindestens einen Stimulationsvorrichtung direkt oder indirekt verbunden ist, und
die mindestens eine Kontrolleinheit mit der mindestens einen MR-Detektionseinheit und dem mindestens einen Magnetfeldsensor direkt oder indirekt verbunden ist, dadurch gekennzeichnet, dass die zwei Magnetfeldsensoren räumlich von einander getrennt platziert sind, und die MR-Detektionseinheit ausgebildet ist aus den von den Magnetfeldsensoren detektierten und an die MR-Detektionseinheit übermittelten Magnetfeldstärken einen räumlichen und/oder zeitlichen Gradienten der Magnetfeldstärken zu bestimmen, und ein MR-Feld zu detektieren und ein MR-Signal an die Kontrolleinheit zu übermitteln, wenn entweder
a) mindestens eine von den mindestens zwei Magnetfeldsensoren detektierten Magnetfeldstärke über einen ersten vorbestimmten Schwellwert liegt, oder
b) die von den mindestens zwei Magnetfeldsensoren detektierten Magnetfeldstärken unterhalb des ersten vorbestimmten Schwellwertes liegt und oberhalb eines zweiten vorbestimmten Schwellwertes liegt und die räumlichen und/oder zeitlichen Gradienten der Magnetfeldstärken in einem ersten vorbestimmten Bereich von Schwellwerten liegen.

Der Gradient bezeichnet dabei die räumliche oder zeitliche Änderung eines Magnetfeldes, wobei die Detektion bei den in einem IMD vorhandenen Magnetfeldsensoren über die Variation der gemessenen Magnetfeldstärken über die Zeit bestimmt werden, da die Magnetfeldsensoren ortsfest in einem jeweiligen IMD oder einer Elektrodenleitung angeordnet sind.

Der Begriff MR-Feld bezieht sich auf das elektromagnetische Feld eines MR-Gerätes, insbesondere eines MR-Gerätes. Besonders charakteristisch ist das statische Magnetfeld eines MR- oder MR-IGerätes, da es besonders stark ist.

Ein Magnetfeldsensor kann insbesondere ein GMR-Sensor, ein Hall-Sensor, ein Reed-Switch, ein MagFET, oder ein anderer bekannter Magnetfeldsensor sein.

In einer bevorzugten Ausführungsform ist die Kontrolleinheit ausgebildet als Reaktion auf das MR-Signal der MR-Detektionseinheit in einen vorbestimmten Betriebsmodus zu wechseln. Insbesondere kann der vorbestimmte Betriebsmodus ein MR-sicherer Zustand oder MR-Mode sein.

Des Weiteren wird bevorzugt, dass das IMD ein implantierbarer Schrittmacher und/oder Defibrillator/Kardioverter (ICD) oder ein IMD zur kardialen Resynchronisation mit einem ICD und/oder Schrittmacher ist.

Auch wird bevorzugt, dass das IMD eine Neurostimulator oder eine Medikamentenpumpe ist.

Bevorzugt wird auch, dass ein MR-sicherer Zustand das Unterdrücken der Abgabe von Hochspannungsschocks und/oder die Bereitstellung alternativer Schrittmacher-Modi, wie temporäres Abschalten der Schrittmacherfunktion (also ein Unterdrücken der Abgabe von Schrittmacherstimulationen) oder das Umschalten in einen asynchronen Stimulationsmodus (Abgabe von Schrittmacherstimulationen ohne Berücksichtigung und/oder Detektion körpereigener Rhythmen) umfasst.

Die Entscheidung über den geeigneten MR-sicheren Zustand kann entweder beim Programmieren des IMD oder automatisch beim Programmieren des IMD oder automatisch bei der Detektion eines MR-Feldes oder automatisch zu vorbestimmten Zeitpunkten oder bei bestimmten Ereignissen oder Patientenzuständen getroffen werden. Bevorzugt ist das IMD durch Programmierung oder durch Fernprogrammierung, "remoteprogramming", in einen MR-sicheren Zustand (MR-Mode) versetzbar.

Auch wird bevorzugt, dass der vorbestimmte Betriebsmodus Parameter enthält, die in Abhängigkeit von den von der MR-Detektionseinheit bestimmten Magnetfeldstärken und/oder zeitlichen Gradientenfeldern und/oder räumlichen Gradientenfeldern vorbestimmbar sind.

Des Weiteren wird bevorzugt, dass wenn die detektierten Magnetfeldstärken unterhalb des ersten vorbestimmten Schwellwertes liegen, die MR-Detektionseinheit grundsätzlich Gradient und Absolutwert der gemessenen Magnetfeldstärken auswertet.

Auch wird bevorzugt, dass die mindestens zwei Magnetfeldsensoren innerhalb des IMD angeordnet sind oder dass mindestens einer der Magnetfeldsensoren innerhalb des IMD angeordnet ist und mindestens ein weiterer Magnetfeldsensor in einer mit dem IMD verbindbaren oder verbundenen Elektrodenleitung angeordnet ist. Eine Elektrodenleitung kann eine Leitung zum Applizieren von elektronischen Impulsen und/oder eine Sensorleitung sein. Im Falle einer Sensorleitung kann der jeweilige Sensor elektronisch, optisch oder optoelektronisch an das IMD angebunden sein.

Im Falle einer Medikamentenpumpe kann die Elektrodenleitung auch eine Hohlleitung zum Applizieren von Medikamenten und/oder eine Sensorleitung sein.

In einer weiteren Ausführungsform wird bevorzugt, dass die MR-Detektionseinheit ausgebildet ist eine vorzeichenbehaftete Differenz der mindestens zwei Magnetfeldsensoren zu bilden, und in Abhängigkeit des Vorzeichens unterschiedliche erste vorbestimmte Schwellwerte und/oder zweite vorbestimmte Schwellwerte und/oder ersten vorbestimmten Bereich von Schwellwerten für die Detektion des MR-Feldes zu verwenden.

Des Weiteren wird bevorzugt, dass der Mindestabstand der mindestens zwei Magnetfeldsensoren entsprechend der Auflösung der mindestens zwei Magnetfeldsensoren so gewählt ist, dass die Auflösung ausreicht um zwischen einem lokalen Magnetfeld und einem weit verteilten MR-Feld an Hand der räumlichen Gradienten zu unterscheiden.

Unter einem lokalen Magnetfeld sind dabei Magnetfelder von Permanentmagneten oder Elektromagneten zu verstehen, wie sie in der normalen Patientenumgebung (z.B. in Form von Lautsprechern oder einfachen Permanentmagneten) vorkommen. Die weit verteilten MR-Felder unterscheiden sich von diesen lokalen Feldern durch ihre schiere Größe. So erstrecken sich die Magnetfelder außerhalb des MR-Gerätes noch 100-150cm mit deutlich messbarer Feldstärke von über 2mT (Ansprechschwelle des Reed-Schalters in üblichen Herzschrittmachern). Die üblicherweise im Patientenumfeld zu erwartenden Magnetfelder erreichen hingegen eine Ausdehnung von wenigen Zentimetern in dieser Feldstärke.

Auch wird bevorzugt, dass ein MR-Signal an die Kontrolleinheit übermittelt wird, die Kontrolleinheit ausgebildet ist mindestens eine vorbestimmte automatische Umschaltung mindestens einer Implantatseinstellung zu bewirken.

In einer weiteren Ausführungsform wird bevorzugt, dass das IMD mindestens eine langgestreckte Elektrodenleitung und/oder mindestens eine Sensorleitung umfasst.

Des Weiteren wird bevorzugt, dass die MR-Detektionseinheit ausgebildet ist eine MR-typische Fahrt eines IMD auf der Patientenliege, basierend auf der zeitlichen Änderung der Messwerte der mindestens zwei Magnetfeldsensoren und/oder der Differenz der Messwerte der mindestens zwei Magnetfeldsensoren, zu erkennen. Unter einer MR-typische Fahrt ist das Einfahren oder das Ausfahren der Patientenliege in das oder aus dem MR-Gerät oder MRI-Gerät mit einem Patienten mit einem IMD zu verstehen. Bei einer solchen Fahrt wird der Patient einer Geschwindigkeit in die oder aus der Scannposition des jeweiligen MR-Gerätes gebracht, die es ermöglicht eine Änderung der Magnetfeldstärken zu bestimmen.

In einer bevorzugten Ausführungsform ist vorgesehen, dass vorbestimmte oder vorbestimmbare Parameter und/oder Schwellwerte und/oder Einstellungen (wie die für einen MR-sicheren Zustand oder MR-Mode oder vorbestimmten Betriebsmodus) über ein lokales Programmiergerät und/oder über Fernprogrammierung eingestellt und/oder verändert und/oder vorbestimmt werden können.

Unter Fernprogrammierung, auch "remote programming" genannt, ist dabei die Programmierung eines IMD zu verstehen, wobei das IMD und die Programmiereinheit, bzw. der Programmierende räumlich so von einander getrennt sind, dass die normalerweise vorhandene Nahfeld-Telemetrie (< 10 m) alleine nicht ausreicht, um die räumliche Trennung zu überbrücken.

In einer weiteren Ausführung wird ein Einschalten, oder das Eingeschaltet sein des MR-sicheren Zustands mittels telemetrischer Fernüberwachung, einem so genannten "Home Monitoring" übertragen. Das heißt, dass beim Einschalten des MR-sicheren Zustandes ein Signal oder eine Information direkt oder über mindestens ein Zwischengerät, z. B. ein Patientengerät, an eine zentrale Einheit gesendet wird, die die Information oder das Signal bearbeiten oder gegebenenfalls weiterleiten. Sollte beim Einschalten des MR-sicheren Zustands keine Verbindung zur zentralen Einheit bestehen oder aufbaubar sein, so wird die Information oder das Signal zu einem späteren Zeitpunkt gesendet oder übertragen, bevorzugt, sobald eine Verbindung zur zentralen Einheit besteht oder diese herstellbar ist.

Telemetrische Fernüberwachung bezeichnet dabei allgemein die Möglichkeit, dass Informationen oder Signale von einem IMD zu einer räumlich entfernten Einheit übertragen werden können, um eine Überwachung des Patienten auch ohne Arztbesuch oder Hospitalisierung zu ermöglichen, indem z. B. Daten aus dem Implantat über ein Service Center an einen zuständigen Arzt übermittelt werden. Prinzipiell kann es auch vorgesehen sein Daten über das Service Center an das IMD zu übermitteln.

Ebenfalls vorteilhaft ist die Kombination mit anderen Verfahren zur Detektion von elektromagnetischen Feldern, speziell MR-Feldern, und Kombination mit unterschiedlichen Reaktionen auf elektromagnetische Felder, speziell MR-Felder. Als weitere Detektionsverfahren sind neben anderen auch:
- GMR-Sensoren,
- Überwachung der Batteriespannung beim Laden eines Kondensators, insbesondere eines Hochspannungskondensators,
- MagFETs,
- Auswertung von induzierten Strömen in Elektroden oder Antennen als Indikator,
- Detektion von spezifischen Vibrationen oder als Sensoren ausgelegte Bauteile zur Detektion von durch Lorentzkräfte induzierten Vibrationen als Indikator und
- Lagesensoren als Indikator vorteilhafte vorsehbar.
Unter Indikatoren sind dabei Verfahren und/oder Vorrichtungen zu verstehen, die Rückschlüsse auf das Vorhandensein von elektromagnetischen Störfeldern zulassen.

Als weitere Reaktionen auf detektierte elektromagnetische Felder, speziell MR-Felder sind neben den bereits genannten auch anderen Reaktionen möglich, wie, aber nicht beschränkt auf,
- ein verlängertes Verbleiben in einem MR-sicheren oder gegenüber elektromagnetischen Störfeldern unempfindlichen Zustand,
- eine Synchronisierung von elektrischen Messungen (z. B. Impedanzmessungen) mit bei periodischen oder gepulsten elektromagnetischen Feldern auftretenden betragsmäßigen Feldstärkenminima oder die Synchronisierung einer Stimulation mit eben jenen betragsmäßigen Minima und
- die Abgabe von elektromagnetischen Pulsen zur Signalisierung, dass ein medizinisches Gerät, speziell ein Implantat, im elektromagnetischen Feld vorhanden ist, speziell zur Signalisierung an ein MR-Gerät, mit der Möglichkeit neben der Störung auch Informationen auf diese Weise zu übertragen und auf dem Bildschirm des MR sichtbar zu machen.

Des Weiteren wird bevorzugt, dass ein Lagesensor zur Plausibilitätsprüfung herangezogen wird und eine positive MR-Erkennung nur erfolgt, wenn der Lagesensor eine liegende Positur und/oder eine andere voreinstellbare Positur meldet.

Insbesondere wird die Kombination des Lagesensors mit der Erkennung einer MRtypischen Fahrt eines IMD auf der Patientenliege bevorzugt, da diese Kombination eine besonders hohe Sensitivität für die Erkennung von MR-Feldern von MR-Geräten bietet.

Besonders bevorzugt ist der Lagesensor selbst kalibrierend, wobei die Kalibrierung unter voreinstellbaren Randbedingungen, wie aber nicht beschränkt auf, Uhrzeiten und/oder Herzraten und/oder Atemfrequenz und/oder hämodynamische Parameter und/oder Aktivität (Bewegungssensor) stattfindet.

Einige Aspekte der Erfindung werden in den Figuren 1 -4 dargestellt.
- Fig. 1: Schematische Darstellung des Ablaufs einer MR-Untersuchung
- Fig. 2: Schematische Darstellung des Verlaufs eines MR B0-Feldes
- Fig. 3: Schematische Darstellung eines Oversensing im MRT
- Fig. 4: Schematische Darstellung eines IMD mit einem 2-Magnetfeldsensorensystem

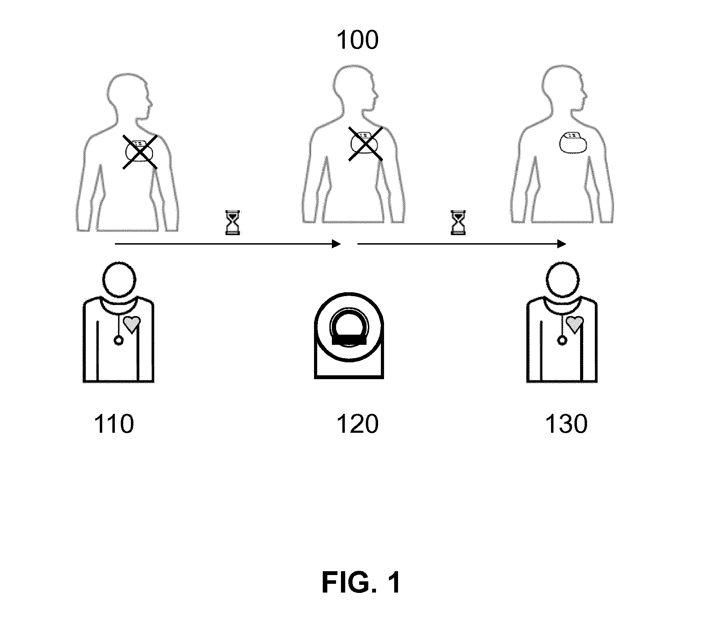

Figur 1 beschreibt den Stand der Technik am Beispiel eines MR-kompatiblen ICD. Der ICD-Patient (100) wird vor der geplanten MRT-Untersuchung von einem Kardiologen nachgesorgt und der ICD ausgeschaltet (110), um eine inadäquate Schockabgabe während des MR-Scans durch Oversensing bedingt durch die auf die Elektrodenleitung wirkenden elektromagnetischen Wechselfelder auszuschließen. Mit einer zeitlichen Verzögerung von Stunden bis Tagen erfolgt die MRT-Untersuchung bei einem Radiologen (120). Nach einer weiteren Verzögerung wird der Patient wieder vom Kardiologen (130) betreut und der ICD wieder eingeschaltet. Während der gesamten Zeit von (110) bis (130) ist der Patient ohne den Schutz des implantierten Defibrillators und weitgehend ohne Rhythmusmonitoring. Derzeit wird dieses verbleibende Restrisiko, gemessen an dem Nutzen der MRT-Untersuchung in Kauf genommen.
Ebenso ist der finanzielle und logistische Aufwand einer solchen Prozedur sehr hoch und schließt in vielen Fällen die Notfallanwendung eines MRT aus.

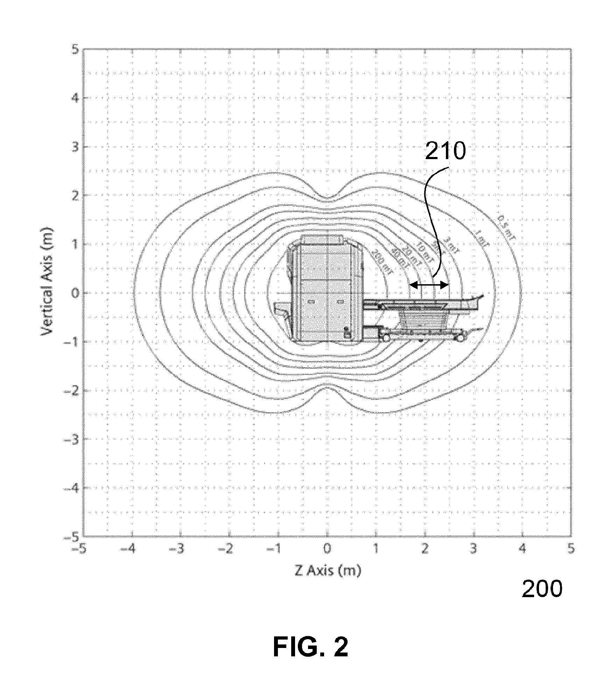

Die Figur 2 zeigt die Problematik bei Verwendung eines einzelnen Magnetfeldsensors zur MRT-Erkennung. Hier ist exemplarisch die Feldstärkeverteilung des B0-Feldes eines MR-Gerätes außerhalb des Scanners aufgetragen (200). Diese Magnetfeldstärke kann bei einem MR-Scan eines Patienten mit den Füßen im Isozentrum an dessen Kopfende bis auf etwa 5mT sinken, so dass ein Magnetfeldsensor in einem Implantat in Kopfnähe keine MR-typische Magnetfeldstärke wahrnehmen kann und so keine automatische MR-Umschaltung durchführt. Das Implantat würde sich dann in entsprechender Entfernung zum Magneten des MR-Gerätes befinden.
Allerdings unterscheidet sich die MR-typische Magnetfeldverteilung durch ihre "Größe" deutlich von der einer "normalen" in der Patientenumgebung zu erwartenden Magnetfeldquelle (z.B. Magnet im Programmierkopf). Hier erreichen die Feldstärken oberhalb von 2mT eine Ausdehnung von wenigen Zentimetern (typischerweise <10cm).

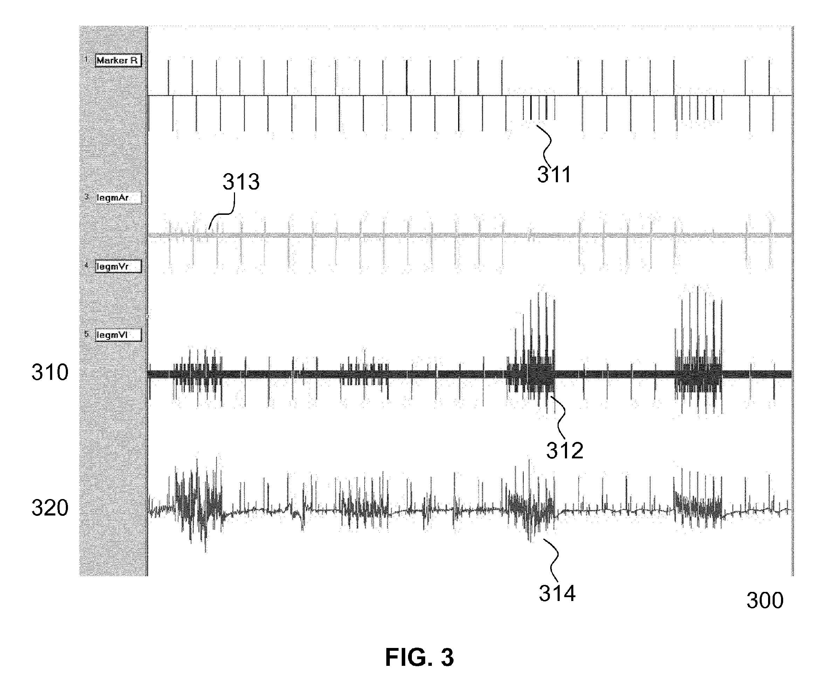

Die Figur 3 zeigt eine mit einem ICD-System durchgeführte Messung (300) in einer der in Figur 2 angenommenen Position eines Patienten mit den Füßen im Isozentrum und einem Implantat im Brustbereich (210). Deutlich ist zu erkennen, dass in den ventrikulären Sensingkanälen eine Oversensing (310, 320) eingeprägt wird, dass im Falle eines nicht deaktivierten ICD zu einer inadäquaten Schocktherapie führen würde. Dies zeigt sich deutlich im Markerkanal an schnellen rechtsventrikulären Detektionsmarkern (311), die in Folge der überschwelligen, eingeprägten MR-Störung (312) vom Implantat wahrgenommen werden. Übersteigt die Anzahl der schnellen Detektionsmarker (311) einen programmierbaren Grenzwert, würde ein ICD eine antitachykarde Therapie inadäquat einleiten.
Vergleichbare Störungen sind auch im rechtsatrialen Sensigkanal (313) und im linksventrikulären Sensingkanal (314) zu sehen. Hier erreichen die Störamplituden jedoch noch nicht die programmierten Wahrnehmungsschwellen, so dass keine falschen Detektionsmarken resultieren.
Das an Implantat zu messende Magnetfeld beträgt hier nur etwas 30mT.

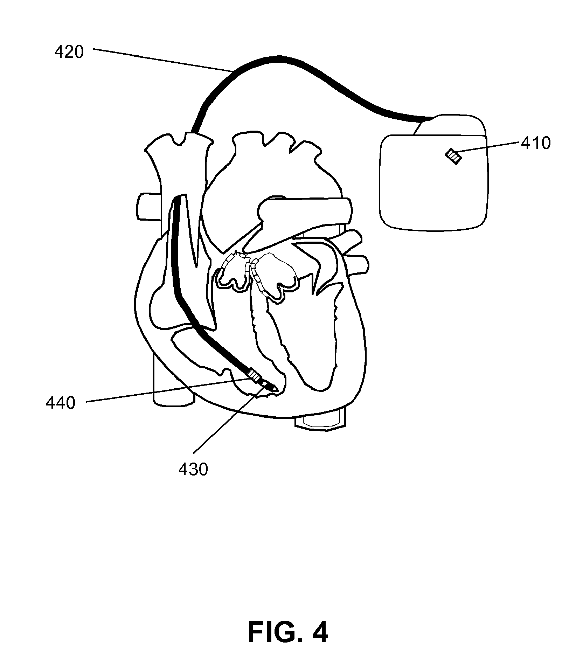

Die Figur 4 zeigt ein IMD-System zwei Magnetfeldsensoren. Das implantierbare medizinische Gerät (IMD) (hier Herzschrittmacher) beinhaltet einen ersten Magnetfeldsensor (410). Das Implantat ist mit einer Elektrodenleitung (420) verbunden, die zur Wahrnehmung und Stimulation des Herzens über einen distalen Dipol (430) ausgelegt ist. Um die räumliche Ausbreitung eines Magnetfeldes erkennen zu können, beinhaltet hier die Elektrodenleitung (420) einen weiteren Magnetfeldsensor (440), dessen Messwerte vom Implantat abgefragt werden können.

Immer dann, wenn der erste Magnetfeldsensor (410) ein überschwelliges Magnetfeld wahrnimmt, wird der zweite Magnetfeldsensor (440) abgefragt und dann eine MRT-Umschaltung durchgeführt, wenn auch der zweite Magnetfeldsensor ein überschwelliges Magnetfeld anzeigt. Die Schwelle des zweiten Magnetfeldsensors wird vom gemessenen Magnetfeld des ersten Magnetfeldsensors abgeleiten und entsprechend den am MRT zu erwartenden Magnetfeldausbreitungen eingestellt. So kann eine sichere Unterscheidung zwischen lokalen Magnetfeldern (wie z.B. Patientenmagneten) oder MR-typischen Magnetfeldern außerhalb des Scanners unterschieden werden.
Überschreitet der Messwert des ersten Magnetfeldsensors eine MR-typische Schwellen (z.B. 100mT), kann auf die Auswertung des zweiten Sensors verzichtet werden.

Optional ist der in der Elektrodenleitung integrierte Magnetfeldsensor (440) vergleichsweise einfach realisiert (Reed-Switch, GMR-Sensor oder ähnliches) und arbeitet mit einer festen, Schwelle (z.B. 5mT). In diesem Fall wird dann bei Aktivierung des Elektrodensensors (440) über den Implantatsensor (410) die MR-Umgebung bestätigt (z.B. Feld > 1mT) oder verworfen (Feld<1mT).

## Patentansprüche

1. Implantierbares medizinisches Gerät (IMD) mit:
mindestens einer Energieversorgung, mindestens einer Abfühlvorrichtung und/oder mindestens einer Stimulationsvorrichtung,
mindestens einer Kontrolleinheit;
mindestens einer MR-Detektionseinheit, und mindestens zwei Magnetfeldsensoren,
wobei
die mindestens eine Energieversorgung mit einem oder mehreren der anderen Komponenten direkt oder indirekt verbunden ist, und wobei
die mindestens eine Kontrolleinheit mit der mindestens einen Abfühlvorrichtung und/oder mindestens einen Stimulationsvorrichtung direkt oder indirekt verbunden ist, und
die mindestens eine Kontrolleinheit mit der mindestens einen MR-Detektionseinheit und dem mindestens einen Magnetfeldsensor direkt oder indirekt verbunden ist,
**dadurch gekennzeichnet, dass** die zwei Magnetfeldsensoren räumlich von einander getrennt platziert sind, und die MR-Detektionseinheit ausgebildet ist aus den von den Magnetfeldsensoren detektierten und an die MR-Detektionseinheit übermittelten Magnetfeldstärken einen räumlichen und/oder zeitlichen Gradienten der Magnetfeldstärken zu bestimmen, und ein MR-Feld zu detektieren und ein MR-Signal an die Kontrolleinheit zu übermitteln, wenn entweder
c) mindestens eine von den mindestens zwei Magnetfeldsensoren detektierten Magnetfeldstärke über einen ersten vorbestimmten Schwellwert liegt, oder
d) die von den mindestens zwei Magnetfeldsensoren detektierten Magnetfeldstärken unterhalb des ersten vorbestimmten Schwellwertes liegt und oberhalb eines zweiten vorbestimmten Schwellwertes liegt und die räumlichen und/oder zeitlichen Gradienten der Magnetfeldstärken in einem ersten vorbestimmten Bereich von Schwellwerten liegen.

2. IMD nach Anspruch 1, **dadurch gekennzeichnet, dass** die Kontrolleinheit als Reaktion auf das MR-Signal der MR-Detektionseinheit in einen vorbestimmten Betriebsmodus wechselt.

3. IMD nach Anspruch 2, **dadurch gekennzeichnet, dass** der vorbestimmte Betriebsmodus Parameter enthält, die in Abhängigkeit von den von der MR-Detektionseinheit bestimmten Magnetfeldstärken und/oder zeitlichen Gradientenfeldern und/oder räumlichen Gradientenfeldern vorbestimmbar sind.

4. IMD nach einem der vorhergehenden Ansprüche, **dadurch gekennzeichnet, dass** wenn die detektierten Magnetfeldstärken unterhalb des ersten vorbestimmten Schwellwertes liegen die MR-Detektionseinheit grundsätzlich Gradient und Absolutwert der gemessenen Magnetfeldstärken auswertet.

5. IMD nach einem der vorhergehenden Ansprüche **dadurch gekennzeichnet, dass** die mindestens zwei Magnetfeldsensoren innerhalb des IMD angeordnet sind oder dass mindestens einer der Magnetfeldsensoren innerhalb des IMD angeordnet und mindestens ein weiterer Magnetfeldsensor in einer mit dem IMD verbindbaren oder verbundenen Elektrodenleitung angeordnet ist.

6. IMD nach einem der vorhergehenden Ansprüche, **dadurch gekennzeichnet, dass** die MR-Detektionseinheit ausgebildet ist eine vorzeichenbehaftete Differenz der mindestens zwei Magnetfeldsensoren zu bilden, wobei in Abhängigkeit des Vorzeichens unterschiedliche erste vorbestimmte Schwellwerte und/oder zweite vorbestimmte Schwellwerte und/oder ersten vorbestimmten Bereich von Schwellwerten für die Detektion des MR-Feldes zu verwenden.

7. IMD nach einem der vorhergehenden Ansprüche, **dadurch gekennzeichnet, dass** der Mindestabstand der mindestens zwei Magnetfeldsensoren entsprechend der Auflösung der mindestens zwei Magnetfeldsensoren so gewählt ist, dass die Auflösung ausreicht um zwischen einem lokalen Magnetfeld und einem weit verteilten MR-Feld an Hand der räumlichen Gradienten zu unterscheiden.

8. IMD nach einem der vorhergehenden Ansprüche, **dadurch gekennzeichnet, dass** in dem Fall, dass ein MR-Signal an die Kontrolleinheit übermittelt wird, die Kontrolleinheit ausgebildet ist mindestens eine vorbestimmte automatische Umschaltung mindestens einer Implantatseinstellung zu bewirken.

9. IMD nach einem der vorhergehenden Ansprüche, **dadurch gekennzeichnet, dass** das IMD mindestens eine langgestreckte Elektrodenleitung und/oder Sensorleitung umfasst.

10. IMD nach einem der vorhergehenden Ansprüche, **dadurch gekennzeichnet, dass** die MR-Detektionseinheit ausgebildet ist eine MR-typische Fahrt eines IMD auf der Patientenliege basierend auf der zeitlichen Änderung der Messwerte der mindestens zwei Magnetfeldsensoren und/oder der Differenz der Messwerte der mindestens zwei Magnetfeldsensoren zu erkennen.
